(19) **Europäisches Patentamt**

**European Patent Office**

**Office européen des brevets**

(11) Veröffentlichungsnummer : **0 180 869**
**B1**

(12) **EUROPÄISCHE PATENTSCHRIFT**

(45) Veröffentlichungstag der Patentschrift :
**17.08.88**

(21) Anmeldenummer : **85113576.4**

(22) Anmeldetag : **25.10.85**

(51) Int. Cl.⁴ : **C 07 D277/80**

(54) **Verfahren zur Herstellung von lagerstabilen Benzothiazolsulfenamiden.**

(30) Priorität : **08.11.84 DE 3440801**

(43) Veröffentlichungstag der Anmeldung :
**14.05.86 Patentblatt 86/20**

(45) Bekanntmachung des Hinweises auf die Patenterteilung : **17.08.88 Patentblatt 88/33**

(84) Benannte Vertragsstaaten :
**AT BE DE FR GB IT NL**

(56) Entgegenhaltungen :
**DE-A- 1 940 365**
**GB-A- 2 053 205**
**JOURNAL OF ORGANIC CHEMISTRY, Band 14, Nr. 6,**
**November 1949, Seiten 921-934, Washington, DC, US;**
**E.L. CARR et al.: "Thiazolesulfenamides"**

(73) Patentinhaber : **BAYER AG**
**Konzernverwaltung RP Patentabteilung**
**D-5090 Leverkusen 1 Bayerwerk (DE)**

(72) Erfinder : **Wassen, Jürgen, Dr.**
**Dürscheider Weg 4**
**D-5090 Leverkusen 3 (DE)**
Erfinder : **Hüllstrung, Dieter, Dr.**
**Friedrich-Bayer-Strasse 11**
**D-5090 Leverkusen 1 (DE)**
Erfinder : **Schlesmann, Harro, Dr.**
**Blecher-Eifgenstrasse 24**
**D-5068 Odenthal (DE)**

## Beschreibung

Die Erfindung betrifft ein Verfahren zur Herstellung von Benzothiazolsulfenamiden, die sich durch verbesserte Reinheit und Lagerstabilität auszeichnen.

Es ist bekannt, Benzothiazolsulfenamide durch gemeinsame Oxidation von Mercaptobenzothiazol (MBT) bzw. von dessen Natriumsalz (NaMBT) und Amin herzustellen (E. L. Carr, G. E. P. Smith, G. Alliger, J. Org. Chem. 14, 921-934 (1949)). Als Oxidationsmittel kommen Chlorbleichlauge, Chlor und Wasserstoffperoxid infrage (C. D. Trivette, E. Morita, E. J. Young, Rubber Chem. Technol. 35, 1374-76 (1962)). Die Reaktion wird dabei aus Gründen der Wirtschaftlichkeit so geführt, daß das Oxidationsmittel so lange zugegeben wird, bis MBT/NaMBT vollständig umgesetzt ist.

Die so mit primären Aminen hergestellten Sulfenamide zeigen eine unbefriedigende Lagerstabilität (J. J. Luecken, M. A. Fäth, Kautschuk + Gummi, Kunststoffe 35, 490-94 (1982)). Um nicht an Wirksamkeit einzubüßen, müssen die Produkte sehr schnell verbraucht und unter besonderen Bedingungen gelagert werden.

Aufgabe der Erfindung ist es, ein verbessertes Verfahren zur Verfügung zu stellen, das zu lagerstabilen Produkten führt. Diese Aufgabe wird überraschenderweise dadurch gelöst, daß man die Oxidation bis zu einem Umsatz des Mercaptobenzothiazols oder seiner Salze von höchstens 95 % durchführt.

Gegenstand der Erfindung ist daher ein Verfahren zur Herstellung von Benzothiazolsulfenamiden aus Mercaptobenzothiazol oder seinen Salzen, insbesondere seinem Natriumsalz und primären Aminen durch Oxidation im wäßrigen Medium und Abfiltrieren, dadurch gekennzeichnet, daß die Oxidation höchstens bis zu einem Umsatz des Mercaptobenzothiazols oder seiner Salze von 95 % durchgeführt wird.

Als primäre Amine kommen bevorzugt Cyclohexylamin, tert. Butylamin, tert. Amylamin und Isopropylamin in Betracht.

Als Oxidationsmittel kommen alle üblicherweise verwendeten Substanzen in Betracht, vorzugsweise Chlorbleichlauge, gasförmiges Chlor und Wasserstoffperoxid.

Das nicht umgesetzte Mercaptobenzothiazol oder seine Salze werden nach der Isolierung des Benzothiazolsulfenamids zurückgewonnen.

Der Umsatz des Mercaptobenzothiazols ergibt sich aus der eingesetzten und der zurückgewonnenen Menge dieser Verbindung.

Der Umsatz beträgt vorzugsweise 80 bis 90 %. Selbstverständlich führen auch geringere Umsätze zu lagerstabilen Produkten, jedoch wird dann das Verfahren zunehmend unwirtschaftlich.

Der Umsatz wird über die Menge des Oxidationsmittels gesteuert. Vorzugsweise setzt man auf 1 Mol Mercaptobenzothiazol oder seiner Salze wenigstens 1 Mol Amin, insbesondere 1 bis 10 Mol, ganz besonders bevorzugt 1,2 bis 5 Mol Amin, und höchstens 1 Äquivalent Oxidationsmittel ein. Die einzusetzende Oxidationsmittelmenge kann nach Art der Reaktionspartner und der Reaktionsbedingungen schwanken, ist aber durch wenige einfache Versuche festzulegen.

Die Reaktion wird vorzugsweise bei einer Temperatur zwischen 20 und 80°C durchgeführt.

Der Gehalt an Wirkstoff wird nach J. G. Lichty, J. O. Cole, A. F. Hardman, R. Leshin, O. Lorenz und C. R. Parks, Ind. Eng. Chem. Prod. Res. Dev. 2, 16 bis 21 (1963) durch Spaltung des Sulfenmids mit $H_2S$ und Titration des frei werdenden Amins mit Säure bestimmt.

Benzothiazolsulfenamide werden in der Gummiindustrie als Vulkanisationsbeschleuniger eingesetzt, die sich durch eine verzögerte Anvulkanisation und eine kurze Ausvulkanisation auszeichnen.

Beispiel 1

N-Cyclohexylbenzothiazolsulfenamid mit Chlorbleichlauge

In einem 3,5 l Planschliffbecher mit Rührer, Thermometer, Rückflußkühler und Tropftrichter wurden

1600 ml Wasser
378 g wäßrige NaMBT-Lösung (50 gew.-%ig)
und 178,2 g Cyclohexylamin
vorgelegt. Unter Rühren gab man innerhalb von 10 Minuten 343 g 20 gew.-%ige Schwefelsäure zu. Dabei heizte sich die Reaktionsmischung auf. Man heizte weiter bis auf 50°C. Bei dieser Temperatur gab man innerhalb von zwei Stunden 457 ml Chlorbleichlauge (Gehalt 150 g NaOCl/l ; Restalkali 5 g NaOH/l) zu. Nach Zulaufende gab man 45 ml 50 %ige NaOH zu und rührte 30 Minuten nach.

Anschließend wurde das Reaktionsgemisch auf eine Temperatur unter 30°C abgekühlt und filtriert. Der Filterrückstand wurde mit Wasser neutral gewaschen und getrocknet.

Man erhielt 223 g N-Cyclohexylbenzothiazolsulfenamid vom Schmelzpunkt 102 bis 103°C mit einem Gehalt von 99 %.

Die Ausbeute bezogen auf Chlorbleichlauge betrug 92 %.

Die Mutterlauge und die ersten drei Waschwässer wurden einer Destillation unterworfen. Man erhielt 0,92 Mol Cyclohexylamin zurück. Die Ausbeute an N-Cyclohexylbenzothiazolsulfenamid bezogen auf umgesetztes Cyclohexylamin betrug damit 96 %.

Der Sumpf der Destillation wurde nach Abkühlen filtriert und dann mit 20 gew.-%iger Schwefelsäure auf pH 2-3 angesäuert. Das ausgefällte MBT wurde abfiltriert, gewaschen und getrocknet. Man erhält 21 g nicht umgesetztes MBT (0,126 Mol) zurück. Die Ausbeute an N-Cyclohexylbenzothiazolsulfenamid bezogen auf umgesetztes MBT betrug damit 97 %.

Zur Untersuchung der Lagerstabilität wurde trockenes Benzothiazolsulfenamid in eine 100 ml Flasche zu 2/3 eingefüllt und mit 1 % Wasser bezogen auf die Einwaage versehen. Dann wurde die Flasche gasdicht verschlossen und 24 Stunden bei 60°C gelagert. Die Abnahme im Gehalt ist ein Maß für die Lagerstabilität. Das unter Beispiel 1 beschriebene Produkt nahm in diesem Test um 1 bis 2 % im Gehalt ab.

Vergleichsbeispiel 1

Führte man die Reaktion, wie unter Beispiel 1 beschrieben, mit 556 ml Chlorbleichlauge (Gehalt 150 g NaOCl/l) durch, so erhielt man 251 g N-Cyclohexylbenzothiazolsulfenamid. Jedoch lag der Schmelzpunkt nur noch bei 99 bis 101°C und der Gehalt bei 96,5 %. Führte man mit diesem Material einen Alterungstest durch, nahm der Gehalt um 17 % ab.

Beispiel 2

N-Cyclohexylbenzothiazolsulfenamid mit Wasserstoffperoxid

In einem 3,5 l Planschliffbecher mit Rührer, Thermometer, Rückflußkühler und Tropftrichter wurden
1600 ml Wasser
378 g wäßrige NaMBT-Lösung (50 gew.-%ig)
und 178,2 g Cyclohexylamin
vorgelegt.
Unter Rühren gab man innerhalb von 10 Minuten 350 g 20 gew.-%ige Schwefelsäure zu. Dabei heizte sich die Reaktionsmischung auf. Man heizte weiter bis auf 50°C. Bei dieser Temperatur gab man innerhalb von zwei Stunden über den getauchten Zulauf 107,7 g Wasserstoffperoxid (30 gew.-%ig) zu. Nach Zulaufende gab man 70 g 50 %ige NaOH zu und rührte 30 Minuten nach.
Der Ansatz wurde wie unter Beispiel 1 beschrieben aufgearbeitet. Man erhielt 211 g N-Cyclohexylbenzothiazolsulfenamid vom Schmelzpunkt 102 bis 104°C und einem Gehalt von 99 %. Bei der Mutterlaugenaufarbeitung wurden 0,96 Mol Cyclohexylamin und 28,8 g nicht umgesetztes MTB zurückgewonnen. Die Ausbeuten bezogen auf $H_2O_2$ waren 84 %, bezogen auf umgesetztes Cyclohexylamin 96 % und auf umgesetztes MBT 97 %. Die Gehaltsabnahme betrug im Alterungstest 1 bis 2 %.

Vergleichsbeispiel 2

Führte man das Beispiel 2 mit 124,7 g 30 gew.-%igem Wasserstoffperoxid durch, erhielt man 241,4 g N-Cyclohexylbenzothiazolsulfenamid. Jedoch war der Schmelzpunkt nur noch 100 bis 102°C, der Gehalt 97 % und die Gehaltsabnahme im Alterungstest 26 %.

Beispiel 3

N-tert.-Butylbenzothiazolsulfenamid mit Chlorbleichlauge

In einem 3,5 l Planschliffbecher mit Rührer, Thermometer, Rückflußkühler und Tropftrichter wurden
1300 ml Wasser
378,0 g wäßrige NaMBT-Lösung (50 gew.-%ig)
und 292,0 g tert. Butylamin
vorgelegt.
Unter Rühren gab man innerhalb von 10 Minuten 588 g 20 %ige Schwefelsäure zu. Dabei heizte sich die Reaktionsmischung auf. Man heizte weiter bis auf 60°C. Bei dieser Temperatur gab man innerhalb von zwei Stunden 472 ml Chlorbleichlauge (Gehalt: 150 g NaOCl/l; Restalkali 5 g NaOH/l) zu. Nach Zulaufende gab man 190 g 50 gew.-%ige NaOH zu und rührt 30 Minuten nach.
Der Ansatz wurde wie unter Beispiel 1 beschrieben aufgearbeitet. Man erhielt 202 g N-tert.-Butylbenzothiazolsulfenamid vom Schmelzpunkt 109 bis 111°C und einem Gehalt von 99 %. Bei der Mutterlaugenaufarbeitung wurden 3,1 Mol tert.-Butylamin und 23,8 g nicht umgesetztes MBT zurückgewonnen.
Die Ausbeute bezogen auf Chlorbleichlauge war 90 %, auf umgesetztes tert.-Butylamin 95 % und auf umgesetztes MBT 99 %. Die Gehaltsabnahme betrug im Alterungstest 1 bis 2 %.

Vergleichsbeispiel 3

Führte man das Beispiel 3 mit 547 ml Chlorbleichlauge (Gehalt 150 g NaOCl/l; Restalkali 5 g NaOH/l) durch, erhielt man 239 g N-tert. Butylaminbenzothiazolsulfenamid. Jedoch war der Schmelzpunkt nur noch 109 bis 110°C, der Gehalt nur noch 98 % und die Gehaltsabnahme im Alterungstest betrug 10 %.

**Patentansprüche**

1. Verfahren zur Herstellung von Benzothiazolsulfenamiden aus Mercaptobenzothiazol oder seinen Salzen und primären Aminen durch Oxidation im wäßrigen Medium und Abfiltrieren, dadurch gekennzeichnet, daß die Oxidation höchstens bis zu einem Umsatz des Mercaptobenzothiazols oder seiner Salze von 95 % durchgeführt wird.

2. Verfahren nach Anspruch 1, dadurch gekennzeichnet, daß Cyclohexylamin, tert. Butylamin, Isopropylamin, tert. Pentylamin als primäre Amine verwendet werden.

3. Verfahren nach Anspruch 1, dadurch gekennzeichnet, daß Chlorbleichlauge, gasförmiges Chlor oder Wasserstoffperoxid als Oxidationsmittel verwendet werden.

4. Verfahren nach Anspruch 1, dadurch gekennzeichnet, daß man mit einem Aminüberschuß arbeitet.

5. Verfahren nach Anspruch 1, dadurch gekennzeichnet, daß der Umsatz des Mercaptobenzothiazols oder seiner Salze 80 bis 90 % beträgt.

6. Verfahren nach Anspruch 1, dadurch gekennzeichnet, daß die Reaktion bei 20 bis 80°C durchgeführt wird.

**Claims**

1. A process for the production of benzothiazolesulfenamides from mercaptobenzothiazole or its salts and primary amines by oxidation in aqueous medium and filtration, characterized in that the oxidation is carried out to a conversion of the mercaptobenzothiazole or its salts of at most 95 %.

2. A process as claimed in claim 1, characterized in that cyclohexylamine, tert.-butylamine, isopropylamine, tert.-pentylamine are used as the primary amines.

3. A process as claimed in claim 1, characterized in that chlorine bleach liquor, gaseous chlorine or hydrogen peroxide are used as oxidizing agents.

4. A process as claimed in claim 1, characterized in that an excess of amine is used.

5. A process as claimed in claim 1, characterized in that the conversion of the mercaptobenzothiazole or its salts comprises 80 to 90 %.

6. A process as claimed in claim 1, characterized in that the reaction is carried out at 20 to 80°C.

**Revendications**

1. Procédé de fabrication de benzothiazolsulfénamides à partir de mercaptobenzothiazol ou de ses sels et d'amines primaires par oxydation en milieu aqueux et séparation par filtration, caractérisé en ce que l'on exécute l'oxydation au plus jusqu'à une conversion du mercaptobenzothiazol ou de ses sels de 95 %.

2. Procédé selon la revendication 1, caractérisé en ce qu'on utilise comme amines primaires de la cyclohexylamine, de la t-butylamine, de l'isopropylamine, de la pentylamine tertiaire.

3. Procédé selon la revendication 1, caractérisé en ce qu'on utilise une solution d'hypochlorite, du chlore gazeux ou du peroxyde d'hydrogène comme agent oxydant.

4. Procédé selon la revendication 1, caractérisé en ce qu'on opère avec un excès d'amine.

5. Procédé selon la revendication 1, caractérisé en ce que la conversion du mercaptobenzothiazol ou de ses sels s'élève à 80 à 90 %.

6. Procédé selon la revendication 1, caractérisé en ce qu'on exécute la réaction à 20 à 80°C.